(19)

Europäisches
Patentamt
European
Patent Office
Office européen
des brevets

(11) **EP 4 224 373 A1**

(12) **EUROPEAN PATENT APPLICATION**

(43) Date of publication:
**09.08.2023 Bulletin 2023/32**

(21) Application number: **22305114.5**

(22) Date of filing: **02.02.2022**

(51) International Patent Classification (IPC):
*G06N 3/04* (2023.01)   *G16H 50/30* (2018.01)
*G16H 50/50* (2018.01)

(52) Cooperative Patent Classification (CPC):
**G16H 50/20; G06N 3/045; G06N 3/08; G16H 50/30;
G16H 50/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Digital for Mental Health**
**75008 Paris (FR)**

(72) Inventors:
• **FOMPEYRINE, Denis**
**75008 PARIS (FR)**
• **PELLEGRIN, Gauthier**
**75008 PARIS (FR)**
• **RICKA, Nicolas**
**75008 PARIS (FR)**

(74) Representative: **Cabinet Nony**
**11 rue Saint-Georges**
**75009 Paris (FR)**

Remarks:
Claims 16-26 are deemed to be abandoned due to
non-payment of the claims fees (Rule 45(3) EPC).

(54) **SYSTEM FOR FORECASTING A MENTAL STATE OF A SUBJECT AND METHOD**

(57)    A computer-based forecasting system for generating a prediction of the evolution of a mental state of a living subject likely to suffer from a mental disorder, the prediction being generated from time-dependent signals acquired beforehand on the subject, the system comprising:

a. A signal processing module for generating, from the time-dependent signals, at least two feature sets comprising time-series of a plurality of extracted features,

b. A hierarchical scoring module comprising at least one predictor comprising at least one ML algorithm for processing the at least two feature sets and generating an evaluation of a respective temporal sub-score, and,

c. A forecasting module comprising a processing module for processing the evaluated temporal sub-scores through at least one ML algorithm and generating a prediction of the evolution of at least one indicator related to the mental state of the subject and/or of the mental state of the subject.

[Fig 1]

Fig. 1

EP 4 224 373 A1

## Description

## Field of the invention

The present disclosure generally relates to systems and methods for generating forecasts in complex dynamical systems such as the evolution over time of a psychological condition, *inter alia.*

## Background of the invention

Machine Learning (ML) has recently been demonstrated to rival expert-level human accuracy in prediction and detection tasks in a variety of domains, including healthcare. The need for automated health care solutions is increasingly gaining interest.

The vision of being able to accurately predict a patient's medical trajectory by integrating vast amounts of disparate data has inspired generations of computer scientists and has resulted in a variety of early applications of artificial intelligence (AI) in the form of decision aids and decision support systems.

Up to recently, the state-of-the-art AI-based forecast in real-life dynamical systems, such as medical-related systems or real-life physical systems, was achieved using recurrent neural networks (RNN). However, these types of neural network may prove difficult to train, especially when it comes to large real-life datasets, which may in return limit the accuracy of the forecast.

Such methods also assume that the data is uniformly sampled, which is rarely the case for real-life systems.

Recently, as described in the paper from Fompeyrine et al. "Enhancing human-machine teaming for medical prognosis through neural ordinary differential equations (NODEs)." (Human-Intelligent Systems Integration, 2021), a specific AI-technology using Neural Ordinary Equations (Neural ODEs) has been shown to successfully reconstruct a real-life dataset comprising intensive care unit (ICU) data of several patients and deliver a forecast in the in-hospital mortality rate of such patients.

However, for some complex systems, real-life time series are often driven by random elements and are sensitive to environmental perturbations; as a consequence, they may follow chaotic, non-easily learnable dynamics.

Moreover, when working with real-life datasets, assessments labels are rarely available at each time step, which makes it difficult to assess the system continuously and to forecast its evolution.

Finally, additional difficulties may arise if the real-life dynamical system to forecast is part of a "at-risk context", where a critical decision is required. In that case, there is a need for the end-user to receive helpful, explainable information from the AI-based system in order to make his decision.

An example of such real-life complex system is the health condition of a patient suffering from a psychological disease such as major depression disorder (MDD) or post-traumatic stress disorder (PTSD).

MDD is a complex disease where the prior art fails to predict critical phases in most cases, as this disorder results in a variety of symptoms whose expression depends on the individual history, capacities, and genetic heritage, and may be influenced by the environment.

There remains a need for improved computerized systems and methods for generating a forecast in time-dependent complex physical systems with a reliable accuracy.

## Summary

The present disclosure aims to remedy to all or part of the deficiencies of the prior art mentioned above and exemplary embodiments of the invention relate to a computer-based forecasting system for generating a prediction of the evolution of a mental state of a living subject likely to suffer from a mental disorder, the prediction being generated from time-dependent signals acquired beforehand on the subject, the system comprising:

> a. A signal processing module for generating, from the time-dependent signals, at least two feature sets comprising time-series of a plurality of extracted features, each feature set relating to a symptom of the mental disorder,
>
> b. A hierarchical scoring module comprising at least one predictor comprising at least one machine learning (ML) algorithm for processing the at least two feature sets and generating, for each feature set, an evaluation of a respective temporal sub-score, and,
>
> c. A forecasting module comprising a processing module for processing the evaluated temporal sub-scores through at least one machine learning (ML) algorithm and generating a prediction of the evolution of at least one indicator related to the mental state of the subject and/or of the mental state of the subject.

One should note that a distinction is made here between "evaluation" and "forecast".

By "evaluation" of an item, it is meant that the item is predicted, or evaluated, on the period of time over which the input data is available.

By "forecast" of an item, it is meant that the item is predicted within a timeframe posterior to the period over which the input data is available. A "forecast" should be understood here as a prediction of a future state, that is, a prediction of the evolution of a state within a timeframe for which data has not been collected yet.

The terms "temporal" or "time-dependent" as used herein mean that information is collected or generated in a successive manner such that a first portion of information is input at a first time, a second portion of information is input at a second time subsequent to the first time, and so on.

"Symptom" refers to a physical or mental sign which is regarded as indicating a condition of disease, particularly such a sign that is apparent to the patient.

### Living subject

A "living subject" refers to any living one for whom the system and methods of the invention may be implemented. Preferentially, the living subject is a human being, though the present disclosure is not limited to humans, but may also include other types of mammals or other animals, such as pets.

By "likely to suffer from a mental disorder", it is meant that the living subject, referred to as a "patient" in some embodiments, has not necessarily been diagnosed as suffering from a mental disorder.

### Mental disorder

The invention is not limited to a particular type of mental disorder.

Preferentially, the mental disorder comprises a mood disorder and/or an anxiety disorder. In particular, the mental disorder may comprise major depression disorder (MDD) or post-traumatic stress disorder (PTSD).

### Acquisition device

Preferentially, the time-dependent signals comprise time series of physiological measurements.

The physiological measurements may comprise one or more among electro-cardiogram (ECG), body temperature, photoplethysmogram (PPG), activity records (ACC), electrodermal activity (EDA).

The system of the invention may comprise an acquisition device sensing the subject for generating said time-dependent signals.

In some embodiments, such an acquisition device comprises a wearable device, such as a wrist monitor, a smart watch, a smart ring, a smart collar, a smart belt, and/or smart patch.

The acquisition device may further comprise other types of smart devices, such as a smart bed, a smart phone, or any other connected device suitable for sensing the subject.

Preferentially, the acquisition device comprises at least one of an accelerometer, an optical sensor, a heart rate detector (e.g., through optical detection or electrical detection) and an electrodermal activity sensor (e.g., through a measurement of dermal electrical impedance or dermal capacity), or electromagnetic sensors such as UWB position sensors.

The acquisition device may comprise one or more cameras for observing attitudes or moves of the subject or parts thereof, and corresponding software for generating based on the images some time-dependent signals.

### Features and feature sets

The plurality of extracted features may comprise data related to actimetry, sleep and wake periods, sleep phases, step counting, cardio-respiratory autonomous nervous system and/or heart rate variability (HRV).

The at least two feature sets are not identical one with the other.

The feature sets may be mutually exclusive, or not. By "mutually exclusive", it is meant that the features do not comprise a common extracted feature.

In some non-limiting embodiments, at least two feature sets comprise at least one common feature.

Preferentially, the system of the invention comprises at least three feature sets, even better at least four feature sets.

In some non-limiting embodiments, the feature sets comprise one or more among sleep quality, anxiety, psychomotor retardation, and diurnal activity, preferably all of them. Said feature sets may comprise certain types of features, for instance:

- the anxiety feature set may comprise features relating to the EDA activity over the subject's sleeping period, and/or activity and heart rate of the subject during a daily period after cessation of social activities,
- the psychomotor retardation feature set may comprise features relating to the activity and/or the heart rate of the subject during the first moments after wake-up,
- the diurnal activity feature set may comprise features relating to the daily number of steps of the subject and/or the daily activity of the subject, and
- the sleep quality feature set may comprise features relating to the activity and/or the sleep phases and/or the sleep and wake periods and/or the heart rate and/or breathing rate of the subject during the night.

Each of these feature sets may be based on one or more of the features listed in the detailed specification below.

### Hierarchical module

Preferentially, the hierarchical module comprises a fusion module for aggregating together at least two evaluated temporal sub-scores and computing a global temporal score representative of the mental state of the subject.

Hence, the at least one indicator related to the mental state of the subject and/or of the mental state of the subject may comprise one or more of the evaluated sub-scores, and/or the global temporal score, depending on the desired output.

By "related to the mental state of the subject", it is meant that the indicator alone may not be sufficient to evaluate the mental state of the subject but may provide information on the mental state of the subject and be useful, for instance taken in combination with other indicators, in the decision-making process of the user of the system of the invention.

By "indicator of the mental state of the subject", it is meant that the indicator alone may provide enough information for the decision-maker and/or the user of the system of the invention to evaluate the mental state of the subject.

Therefore, one or more of the evaluated sub-scores may be used as an indicator related to the mental state of the subject while the global temporal score may be used, either alone or in combination with one or more of the evaluated sub-scores, as an indicator of the mental state of the subject.

Preferentially, the at least one machine learning algorithm of the predictor comprises an explainable machine learning algorithm comprising one or more among logistic regression, shallow multi-layer perceptron or Neural 2-Choquet integrals.

By "explainable", it is meant that the algorithm's internal computation, that is, the obtention of its output from inputs, is readable by the man skilled in the art, either directly, or through extra-information provided by the system along with its output. The man skilled in the art is for example a clinician expert of the mental disorder from which the subject is likely to suffer.

In other words, the explainable ML algorithm of the invention is transparent enough that the man skilled in the art may understand how the prediction of the temporal sub-scores is computed.

In some non-limiting embodiments, the at least one ML algorithm of the predictor comprises a Neural 2-Choquet integrals network, as described in the paper from Bresson, Roman, et al. "Neural representation and learning of hierarchical 2-additive Choquet integrals." (Proceedings of the Twenty-Ninth International Conference on International Joint Conferences on Artificial Intelligence. 2021), the content of which is incorporated herein by reference.

Preferentially, the at least one ML algorithm of the hierarchical scoring module is trained beforehand using a training dataset collected from multiple living subjects.

Exemplary embodiments of such a training method are described below in more details.

## Forecasting module

The invention is not limited to a specific type or architecture for the ML algorithm.

However, some types may be particularly well adapted for processing temporal data.

Preferably, the at least one ML algorithm of the forecasting module comprises a neural network (NN). Said NN may comprise Neural ODEs (NODEs), a temporal one-dimensional convolutional neural network (1D CNN), a recurrent neural network (RNN), or a combination thereof.

The NN may be a RNN of long short-term memory (LSTM) or Gated Recurrent Unit (GRU) type. It may be bi-directional, or not.

Preferentially, the at least one ML algorithm of the forecasting module comprises Neural ODEs (NODEs).

Preferentially, the at least one ML algorithm of the forecasting module is configured to generate a forecast for each temporal sub-score.

The forecasting module may further comprise a fusion module for aggregating together the forecasts of the sub-scores and computing the forecast of a global score representative of the mental state of the subject.

In some non-limiting embodiments, the processing module comprises:

- an encoder for building a point or a distribution of probability in a latent space using the temporal sub-scores, and
- neural ODEs predicting the evolution of said point or said distribution of probability and/or the evolution of the temporal sub-scores using an ordinary differential equation.

The encoder and the NODEs may be trained simultaneously and beforehand using data previously acquired on the subject.

The encoder may use the temporal sub-scores with existing features and/or additional features.

The forecasting module may further comprise a decoder for transforming the predicted evolution of said point or said distribution of probability into predictions of the evolution of the temporal sub-scores.

The NODEs may be trained using ELBO loss function, as described in the publication from Rubanova, Yulia et al. "Latent odes for irregularly-sampled time series." (arXiv preprint arXiv:1907.03907 (2019)), the content of which is incorporated herein by reference.

## User-interface

In certain non-limiting embodiments, the system of the invention may further comprise a user-interface for informing a user of the at least one indicator related to the mental state of the subject and/or of the mental state of the subject.

The term "user" as used herein includes, for example, a person or entity that owns a user computer, such as a computing device or a wireless device; a person or entity that operates or utilizes a user computer; or a person or entity that is otherwise associated with a user computer. It is contemplated that the term "user" is not intended to be limiting and can include various examples beyond those described.

The user may be a different person from the living subject. The user may be a decisionmaker of various nature. For instance, the user may be a clinician and the living subject a patient of the clinician. The user may be the subject himself. As a variant, the user may be a commander, supervisor, a manager, or an owner of the subject (human or animal).

Method to generate a prediction of the evolution of the mental state of a subject

Another aspect of the present invention relates to a computer-implemented method for forecasting the evolution of a mental state of a living subject likely to suffer from a mental disorder, the method comprising:

a) Acquiring during a monitoring period between a first and a second date time-dependent signals from at least one acquisition device sensing the subject,
b) Generating, preferably using a system as defined above, at least two feature sets comprising time-series of a plurality of features extracted from these signals, each feature set relating to a symptom of the mental disorder,
c) Using at least one machine learning algorithm for generating, for each feature set, an evaluation of a respective temporal sub-score between said first and second date,
d) Using at least one machine learning algorithm to process the evaluated temporal sub-scores and generate a prediction of the evolution of a least one indicator related to the mental state of the subject and/or of the mental state of the subject.

By "computer-implemented", it is meant that the method is performed by an electronic hardware equipment such as a personal computer, server, microcontroller board or similar devices, with the addition if needed of the required Human-Machine interfaces (HMI) and signal processors, as described below.
In some non-limiting embodiments, the method further comprises computing at step c), with the at least one ML algorithm, a global temporal score from the temporal sub-scores between said first and second date, the global score being representative of the mental state of the subject between said first and second date.
Preferentially, the at least one ML algorithm for processing the temporal sub-scores comprises a neural network. Such neural network may be a neural ODE, a temporal one-dimensional convolutional neural network (1D CNN), a recurrent neural network (RNN) or a combination thereof, more preferentially a neural ODE.
Preferentially, the method comprises generating with the at least one neural network at step d) a forecast for each temporal sub-score.
The forecasts of the sub-scores may then be aggregated to compute the forecast of a global score as an indicator of the mental state of the subject.
The aggregation may be conducted with a ML algorithm, such as a neural network, or any other appropriate computer-based method. Such ML algorithm may be the same as the ML algorithm used for processing the temporal sub-scores or may be a different one.
The aggregation may be conducted using an aggregation formula learned by a ML algorithm during training, for example the same aggregation formula learned by the above-mentioned ML algorithm of the hierarchical module.
In some non-limiting embodiments, the method comprises at step d), encoding a point or a distribution of probability in a latent space representative of interactions between the symptoms from the sub-scores before processing them with the at least one machine learning algorithm.
In some non-limiting embodiments, the method further comprises encoding a gaussian distribution probability in the latent space to draw multiple latent points from it, or encoding a latent point and perturbing it, to estimate the probability for an event related to the mental disorder to occur in a given time frame.
Such an approach may be useful to estimate the probability for critical events related to the mental disorder to occur in the future; for example, the probability for a subject of being in remission within a particular time frame may be evaluated using this method. The perturbations may also be useful for estimating the disorder recurrence or relapse for a particular patient.

Time periods

The present invention is not limited to a particular monitoring period, or to a particular time period over which the prediction of the evolution is generated.
"Monitoring period" refers to the period over which data is used for the evaluation of the temporal sub-scores, and eventually, the forecasts of the sub-scores.
The acquisition device may collect and/or store data continuously but it may be chosen to evaluate the temporal sub-scores based on a monitoring period shorter than the actual time period over which data is available.
The monitoring period may range from a couple of hours to a couple of days. The duration may be shorter or longer if possible or needed. Such a time period offers enough data for an accurate evaluation of the sub-scores, while staying short enough to limit the data storing and processing cost.
The time-dependent signals are preferentially collected at a frequency between from 0,1Hz to 300 Hz, better between 1Hz and 250 Hz.
The time-dependent signals may be collected at different sample rates, depending for example on the acquisition device used for the measurements. Some signals may be collected at high frequency, such as the PPG measurements, the activity records or EDA. By "high frequency", it is meant that the sample frequency of the data is large enough to allow observation of the physiological signal itself.
PPG measurements may be obtained using a high frequency of at least 40 Hz, or more, or less.

The activity records may be collected at a frequency of about 25Hz, or more, or less.

The EDA measurements may be collected at a frequency of about 4Hz, or more, or less.

The ECG measurements may be collected at a frequency of about 250Hz, or more, or less.

In some non-limiting embodiments, the plurality of features is extracted at a frequency rate from the signals ranging from hourly to daily.

In some non-limiting embodiments, the prediction of the evolution of a mental state of the subject is being generated over a time period of at least 24 hours following the monitoring period, preferentially at least 3 days, or even more, for example one month or more.

Computer System

By "computer-based", it is meant that the above-described system and method according to the invention may be implemented using any computer system.

The computer system may include any automated system in the form of any hardware and/or software, such as a laptop, a personal computer, a smartphone, a workstation, a computer terminal, one or several network computers, or any other data processing system or user device.

Preferentially, the computer system comprises at least one processing unit which controls the overall operation related to the method of the invention by executing computer program instructions. The processing unit may comprise general or special purpose microprocessors, microcontrollers or both, or any other kind of CPU, GPU, FPGA, quantum processor and any combination of those....

The computer system may comprise one or more storage media on which computer program instructions and/or data are stored. The storage media may comprise magnetic, magneto-optical disks, optical disks, flash memory devices, solid state drives (SSD), cloud storage, or any other type of removable storage medium or a combination of both.

The computer system preferably comprises at least one memory unit that can be used to load the instructions or/and data when they are to be executed by the processing unit.

The memory unit comprises for instance a random-access memory (RAM) and/or a read only memory (ROM).

The computer system may further comprise one or more user interfaces, such as any type of display, a keyboard, a pointing device such as a mouse or trackpad, audio input devices such as speakers or microphones, or any other type of device that allows user interaction with the computer system.

The computer system may comprise one or more network interfaces for communicating with other devices.

Another aspect of the invention relates to a computer program product comprising code instructions that cause a computer system to perform the method of the invention when the program is run on the computer system.

In particular, another aspect of the invention relates to a computer program product which, when the program is executed by a computer, cause the computer to carry out a method comprising:

　a) Generating, preferably using a system as defined above, from time-dependent signals acquired beforehand on a living subject likely to suffer from a mental disorder, at least two feature sets comprising time-series of a plurality of features extracted from these signals, each feature set relating to a symptom of the mental disorder,

　**b)** Using at least one ML algorithm for generating, for each feature set, an evaluation of a respective temporal sub-score between said first and second date,

　**c)** Using at least one ML algorithm to process the evaluated temporal sub-scores and generate a prediction of the evolution of a least one indicator related to the mental state of the subject and/or of the mental state of the subject.

Training method

Another aspect of the invention relates to a method for training a ML algorithm, notably a neural network, preferentially a ML algorithm used in a system as defined above, to compute at least two temporal sub-scores for the evaluation of a mental state of a living subject likely to suffer from a mental disorder, each temporal sub-score being an indicator of the state of a symptom of the mental disorder, the method comprising:

- Generating a learning dataset using data obtained from a plurality of living subjects through, for each subject:

　○ acquiring during a monitoring period, time-dependent signals from at least one acquisition device sensing the subject,
　○ generating at least two feature sets comprising time-series of a plurality of features extracted from these signals, each feature set being associated to a respective temporal sub-score to be evaluated,
　○ prompting a user to input clinical evaluation labels relating to the clinical evaluation of the mental state of said subject at respective visit dates during the monitoring period, and
　○ using a ML algorithm to generate pseudo-evaluation labels at additional dates between the visit dates, each representative of an assessed mental state of the subject at a corresponding additional date,

- Using said learning dataset for training the ML algorithm, notably the neural network, to estimate, for each subject, the temporal sub-scores over the monitoring period,

   the ML algorithm being trained simultaneously to aggregate the temporal sub-scores into a temporal global score over the monitoring period, said joint training using a performance criterion based on a comparison between said temporal global score with the pseudo-evaluation labels.

The clinical evaluation labels are preferentially subject assessments obtained using a clinical scale. The clinical scale may be known from the art for the mental disorder considered.

Preferentially, the plurality of features is extracted at a frequency rate from the signals and the pseudo-evaluation labels are generated at a same frequency rate as the frequency rate of plurality of features.

In some non-limiting embodiments, the pseudo-evaluation labels may be generated on a monthly basis.

The ML algorithm generating the pseudo-labels may comprise a neural network such as a deep neural network, for instance a self-supervised neural network.

As mentioned above, the ML algorithm to be trained may comprise a hierarchical 2-Choquet integral network.

System and method for generating a prediction of the evolution of the health condition of a living subject

A further aspect of the present invention, taken in combination or independently from the above, relates to a computer-based forecasting system for generating a prediction of the evolution of the health condition of a living subject likely to suffer from a health disorder, the prediction being generated from time-dependent signals acquired beforehand on the subject, the system comprising:

   a. A signal processing module for generating, from the time-dependent signals, at least two feature sets comprising time-series of a plurality of extracted features, each feature set relating to a symptom of the health disorder,
   b. A hierarchical scoring module comprising at least one predictor comprising at least one machine learning algorithm for processing the at least two feature sets and generating for each feature set, an evaluation of a respective temporal sub-score, and,
   c. A forecasting module comprising a processing module for processing the evaluated temporal sub-scores through at least one machine learning algorithm and generating a prediction of the evolution of at least one indicator related to the health condition of the subject and/or of the health condition of the subject.

Another aspect of the present invention, taken in combination or independently from the above, relates to a computer-implemented method for forecasting the evolution of a health condition of a living subject likely to suffer from a health disorder, the method comprising:

   a) Acquiring during a monitoring period between a first and a second date time-dependent signals from at least one acquisition device sensing the subject,
   b) Generating, preferably using a system as defined above, at least two feature sets comprising time-series of a plurality of features extracted from these signals, each feature set relating to a symptom of the health disorder,
   c) Using at least one machine learning algorithm for generating, for each feature set, an evaluation of a respective temporal sub-score between said first and second date,
   d) Using at least one machine learning algorithm to process the evaluated temporal sub-scores and generate a prediction of the evolution of a least one indicator related to the health condition of the subject and/or of the health condition of the subject.

The features of the systems and methods described above apply to the system and method of this section, in combination or independently from one another.

**Brief description of the drawings**

For a more complete understanding of the present invention, a description will now be given of several examples, taken in conjunction with the accompanying drawings, in which:

   Fig.1 is a partial and schematic representation of an exemplary embodiment of a system in accordance with the invention,
   Fig.2 is a partial and schematic representation of an exemplary embodiment of a signal processing module in accordance with the invention,
   Fig.3 shows an example of time-dependent signals acquired on a patient,
   Fig.4 is a graph showing exemplary temporal sub scores evaluated by a hierarchical scoring module according to the invention,
   Fig.5 is a graph showing an exemplary global score computed by a hierarchical scoring module according to the invention,
   Fig.6 is a partial and schematic representation of an exemplary training method of the neural network of the hierarchical scoring module in accordance with the invention,
   Fig.7 is a graph showing an exemplary reconstruction of the temporal pseudo-labels and the evaluated global score in one embodiment of the invention,

Fig.8 is a partial and schematic representation of an exemplary embodiment of the forecasting module in accordance with the invention,

Fig.9 is a set of four graphs showing an exemplary forecast of sub-scores generated by the processing module of the forecasting module of figure 8, and

Fig.10 is a graph showing an exemplary forecast of global score generated by the forecasting module of figure 8.

## Detailed description

The terms used in this specification generally have their ordinary meanings in the art, within the context of this disclosure and in the specific context where each term is used. Certain terms are discussed below, or elsewhere in the specification, to provide additional guidance in describing the compositions and methods of the disclosure and how to make and use them.

As used herein, the words "a" or "an," when used in conjunction with the term "comprising" in the claims and/or the specification, can mean "one," but they are also consistent with the meaning of "one or more," "at least one," and/or "one or more than one." Furthermore, the terms "having," "including," "containing" and "comprising" are interchangeable, and one of skill in the art will recognize that these terms are open ended terms.

The terms "about" or "approximately" mean within an acceptable error range for the particular value as determined by one of ordinary skill in the art, which will depend in part on how the value is measured or determined, i.e., the limitations of the measurement system.

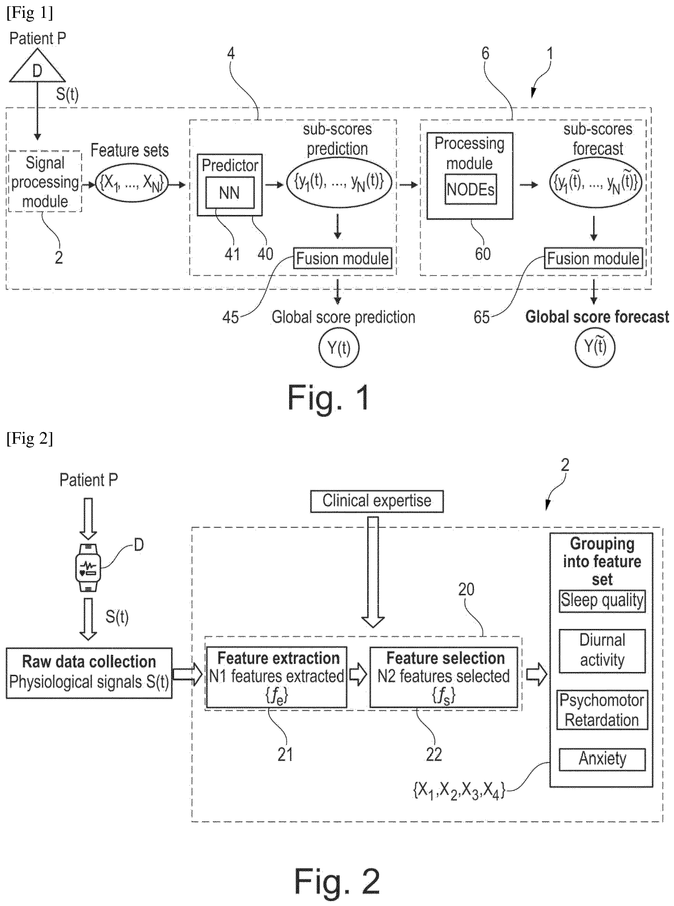

An example of a system 1 according to the invention is shown in Figure 1.

The system 1 comprises a signal processing module 2, which generates N features sets $\{X_1(t),...,X_N(t)\}$ from time-dependent signals S(t). The time-dependent signals S(t), also referred to as also referred as "raw measurements" or "raw data" in the following, are acquired over time on a subject P likely to suffer from a mental disorder.

The time-dependent signals S(t) may be acquired on the subject using a wearable device D comprising one or more sensors, for instance a wrist monitor, a smart patch, or any other device suitable to collect the desired data.

The feature sets $\{X_1(t), ...,X_N(t)\}$ are provided as an input to a hierarchical scoring module 4. A feature set $X_i(t)$ (for $i$ between 1 and N) is a vector or a matrix comprising one or more time-series of the extracted feature.

The hierarchical scoring module 4 comprises a predictor 40, which generates for each feature set $X_i(t)$ a prediction of a sub-score $y_i(t)$. The predictions $\{y_1(t), ..., y_N(t)\}$ are real-time predictions and not forecasts, meaning, they are evaluated at a time or over a period of time on which raw data S(t) has been collected.

The hierarchical scoring module 4 may further comprise a fusion module 45 which computes a prediction of a global score $Y(t)$ from the sub-scores' predictions $\{y_1(t), ...,y_N(t)\}$, over the same period of time as the sub-scores.

The global score $Y(t)$ may be considered as an indicator of the mental state of the subject at time t.

The predictor 40 may comprise, as illustrated in figure 1, a ML algorithm such as neural network 41 to process the features sets and evaluate the sub-scores. The features sets $\{X_1(t), ..., X_N(t)\}$ may be processed together by the predictor 40, or independently.

The neural network 41 may present an architecture known in the art to perform hierarchical scoring tasks. Such neural network is for instance a Neural 2-Choquet integrals network as described in the paper from Bresson, Roman, et al. "Neural representation and learning of hierarchical 2-additive Choquet integrals." (Proceedings of the Twenty-Ninth International Conference on International Joint Conferences on Artificial Intelligence. 2021).

As a variant or in combination with the above, the ML algorithm of the predictor may comprise logistic regressions or shallow multi-layer perceptron.

The fusion module 45 may also comprise a ML algorithm, which may, or, not, be the same as the one of the predictor.

The ML algorithm preferentially comprises an explainable machine learning algorithm. As described above, by "explainable", it is meant here that the fusion of the sub-scores into the global score should be explainable for a user, such as a clinical expert. Hence, an explainable machine learning algorithm gives transparency in its processes, including the process of delivering interpretable results. For instance, the fusion module may learn an explicit aggregation formula to compute the global score $Y(t)$.

Preferentially, the ML algorithms of the predictor 40 and of the fusion module 45 are trained together so that the hierarchical scoring module learns to compute both the sub-scores and the aggregation formula for computing the global score Y(t).

During training, the hierarchical scoring module 2 may use temporal pseudo-labels E(t) as an additional input to learn to compute the sub-scores and the aggregation formula.

Pseudo-labels E(t) are time-series of the same frequency, or time intervals between two consecutive values, as the extracted features. Pseudo-labels E(t) are obtained from a finite number P of empirical assessments $\{E_1,..., E_P\}$, also referred as "empirical labels", sparsely collected on the patients over the same period of time as the time-dependent signals S(t).

The empirical assessments $\{E_1, ...,E_P\}$ may be patient assessments obtained using a clinical scale.

Pseudo-labels E(t) may be generated from the empirical assessments $\{E_1, ...,E_P\}$ using a ML algorithm that propagates the empirical labels so that values are generated regularly over the entire period of time.

The global score estimated by the algorithm is then compared to the pseudo-labels, either by an error between the predicted value and the pseudo-label value or by comparing the evolutions of the two curves using conventional methods (ranking loss, correlation coefficients, DTW distance, ...).

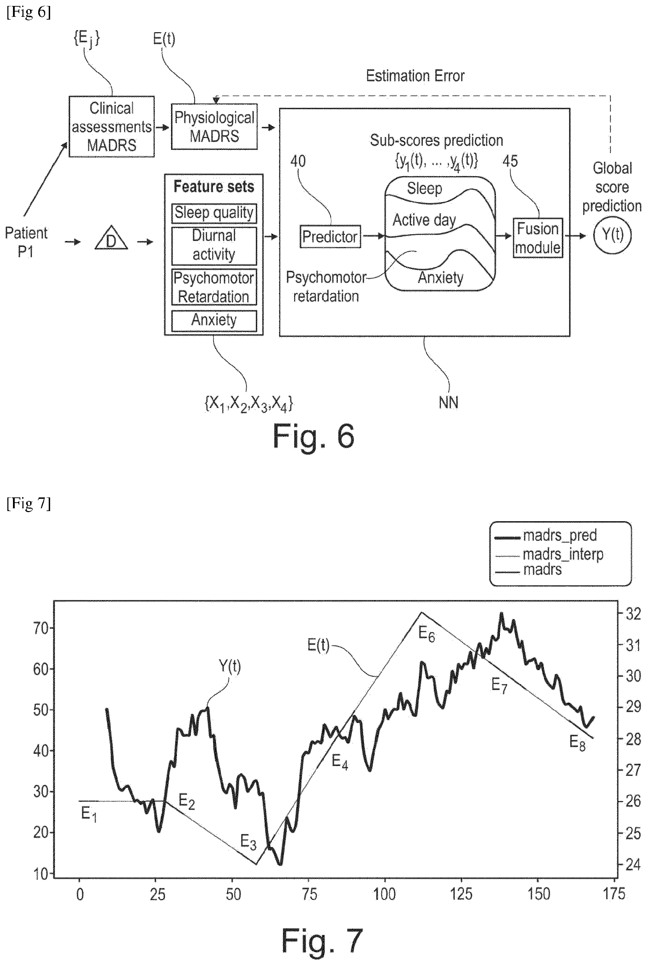

An exemplary training scheme is described in more details below for one exemplary embodiment of the invention, with reference to figures 6 and 7.

The system 1 according to the invention further comprises a forecasting module 6 to generate a prediction of the evolution of the sub-scores $\{y_1(t), ..., y_N(t)\}$. The forecasting module comprises a processing module 60 taking as input the evaluated sub-scores $\{y_1(t), ..., y_N(t)\}$ and generating a forecast $y_i(\tilde{t})$ for each the sub-scores, the notation "$\tilde{t}$" referring to a time in the future.

In general, as described earlier, the processing module 60 comprises a ML algorithm learning the dynamics of the system.

In the example considered, the processing module 60 comprises a Neural ODEs, which is a neural network 61 learning an ordinary differential equation (ODE). In other embodiments, the processing module 60 may comprise other types of ML algorithms, such as a RNN or 1D-CNN

In the example considered, the N forecasted sub-scores $\{y_1(t), ..., y_N(\tilde{t})\}$ are then aggregated by a fusion module 65 into a forecasted global score $Y(\tilde{t})$, said global score $V(\tilde{t})$ being representative of the mental state of the patient at time $\tilde{t}$, that is, of the evolution of the mental state of this patient in the future.

The N forecasted sub-scores $\{y_1(\tilde{t}), ..., y_N(\tilde{t})\}$ may directly be usable by a decision-maker, such as a clinician, to understand the dynamics of the mental disorder.

The N forecasted sub-scores $\{y_1(t), ..., y_N(\tilde{t})\}$ and/or the global score $Y(\tilde{t})$ may be displayed on an interface to the decision-maker, if desired.

As described earlier, the system 1 according to the invention may be configured for monitoring and predicting the evolution of the mental state of patients suffering from a mental disorder.

In particular, the system 1 may be applied with success to the forecast of the mental state of patients suffering from major depression disorder (MDD) or post-traumatic stress disorder (PTSD).

As described above, the dynamics of the progression of MDD is highly complex and still poorly understood.

An exemplary system for forecasting a health condition of a patient suffering from MDD will now be described with reference to figures 2 to 11.

### ➢ Data collection and feature selection

As illustrated in figure 2, the raw signals S(t) are physiological measures collected on a patient P using a wearable device such as a wrist monitor D equipped with multiple sensors.

In the example considered, the signals S(t) are extracted from the electrical signals generated by the sensors of the device D using classical methods, and processed into three high frequency time-dependent variables:

- a photoplethysmogram (PPG), which represents the changes blood volume changes in the microvascular bed of tissue of the patient,
- electrodermal activity (EDA), which refers to the variation of the electrical conductance of the skin in response to sweat secretion, and
- activity record (ACC) which refers to acceleration data obtained from an accelerometer.

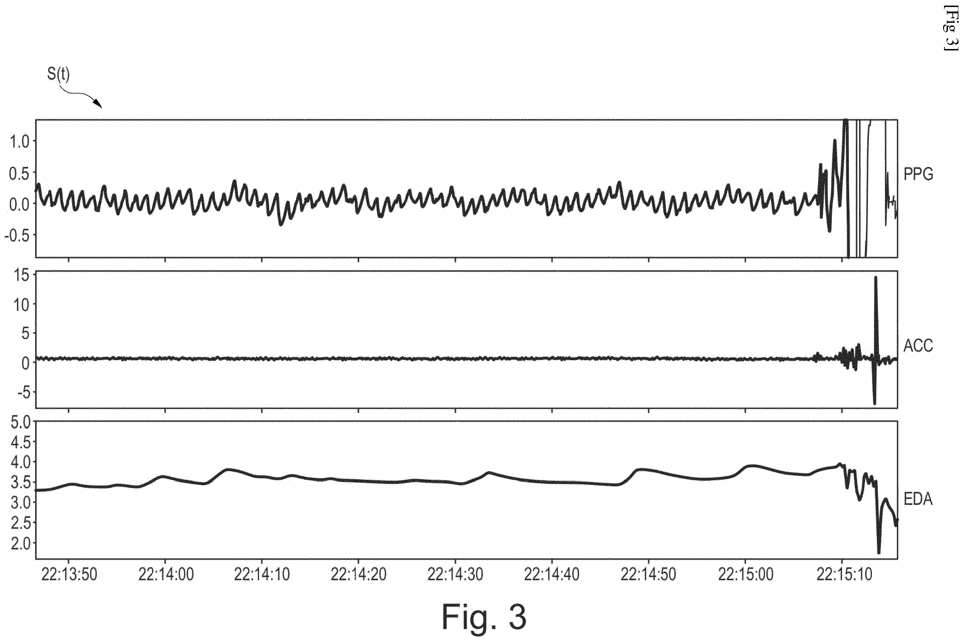

Examples of such signals S(t) are represented in figure 3 over a short period of time.

The device may be continuously worn as in ambulatory mode, or not.

### Features extraction

As shown in figure 2, the signal processing module 2 may comprise a feature generator 20 which extracts features from the signal S(t) through a feature extraction module 21 and selects a limited number of relevant features through a feature selector 22.

The extracted features $\{f_e\}$ may comprise high-frequency sampling of heart rate (HR), breathing rate (BR), multiple indicators of heart rate variability and actimetry features.

The extraction of the features may be conducted through algorithms known in the art.

The extracted features are for example based on activity levels (actimetry), sleep and wake periods, sleep phases, and an estimation of the number of steps.

The activity level may be estimated based on a scale of intensity comprising four levels, namely Sedentary, Low, Moderate and Vigorous, as described in the paper from Menai, Mehdi, et al. "Accelerometer assessed moderate-to-vigorous physical activity and successful ageing: results from the Whitehall II study." (Scientific reports 7.1 (2017): 1-9.).

Sleep and wake periods may be determined using algorithms known in the art for distinguishing the physiology of the patient while he is asleep from his physiology during the day. For example, a subject-specific Hidden Markov Model may be used to discriminate between wake and sleep, which is particularly well fitted for datasets comprising patients with potentially disturbed and various sleep patterns.

The different sleep phases during the patient's sleep may be determined based on the relationship between heart rate variability and sleep phases, as described in Otzenberger, Helene, et al. "Dynamic heart rate variability: a tool for exploring sympathovagal balance continuously during sleep in men."(American Journal of Physiology-Heart and Circulatory Physiology 275.3 (1998): H946-H950.), in Thomas, Robert Joseph, et al. "An electrocardiogram-based technique to assess cardiopulmonary coupling during sleep." (Sleep 28.9 (2005): 1151-1161.), or in Roebuck, A., et al. "A review of signals used in sleep analysis." (Physiological measurement 35.1 (2013): R1).

A classification algorithm adapted from methods described in the above-mentioned papers or other methods known in the art may be used to label measured time windows of sleep as REM sleep, wake, or light/deep sleep.

The step counting may be performed using characteristic oscillations induced by walk on the accelerometer data. This count is for example divided into 2 steps: first a convolutional neural network is trained to detect the walking periods, and then the number of steps is estimated by counting the significant acceleration peaks in the accelerometer signal.

Features selection

From the pool of extracted physiological features $\{f_e\}$, relevant features $\{f_s\}$ may be selected by the feature selector 22. The selection process is preferentially carried out beforehand because it is the same for all patients with major depression disorder.

The selection may be based on specific knowledge of the mental disorder; for example, the features may be selected by a clinician with knowledge of the symptoms that are characteristic of MDD.

As a variant or in combination with expert's knowledge, the features $\{f_s\}$ may be selected using a correlation analysis between the features and empirical data $\{E_j\}$ collected on the subject.

In the example illustrated in figure 2, empirical data comprise clinical assessments collected during clinical visits, such as, for MDD, a score on the Montgomery-Asberg Depression Rating Scale (hereafter denoted as MADRS) assessed by the clinician on the day of the visit.

The MADRS score is a value on a scale from 0 to 60 determined based on a clinical interview, reflecting the psychological state of the patient on that day, in which the disease's severity is classified in 4 categories:

- healthy: MADRS between 0 and 6,
- low: MADRS between 7 and 19,
- intermediate: MADRS between 20 and 34, and
- severe: MADRS between 35 and 60.

Such empirical assessments generally constitute sparse and irregular data over time. However, a timeframe of validity may be defined for these clinical assessments, also referred to as "clinical labels" in the following.

By "validity", it is meant here that the clinical assessment is actually representative of the physiology of the patient.

The timeframe of validity may be of various length depending on the application; in the example considered, the given value of MADRS is determined valid in a timeframe of 5 days before and after the visit.

A correlation indicator CORR may be computed for each extracted feature $f_e$ by comparing the features to the clinical assessment over their respective timeframe of validity. The correlation indicator CORR may participate in the selection process of the relevant features $\{f_s\}$.

In the example considered, the following 34 features $\{f_s\}$ are selected from 137 extracted physiological features $\{f_e\}$:

- *fhawo_LAI:* fraction of the moments when the patient's activity intensity is categorized as Low Activity Intensity, over the first hour after wake onset. The first hour after wake onset is detected by the wake/sleep discriminator as described above.
- *Fhawo_MAI:* fraction of the moments when the patient's activity intensity is categorized as Moderate Activity Intensity, over the first hour after wake onset,
- *Fhawo_VAI:* fraction of the moments when the patient's activity intensity is categorized as Vigourous Activity Intensity, over the first hour after wake onset,
- *fhawo_hr_high:* value of the top decile of Heart Rate values over the first hour after wake onset,
- *fhawo_hr_high_regularity:* standard variation of the fhawo_hr_high over multiple days or weeks, for instance 21 days,
- *n_steps:* daily total number of steps of the patient,
- *n_steps_regularity:* standard variation of n_steps over multiple days or weeks, for instance 21 days,
- *sedentary_day:* fraction of the moments when the activity intensity of the patient is categorized as « sedentary » (no to little activity) during the diurnal portion of the day, that is, when the patient is awake,
- *LAI_day:* fraction of the moments when the activity intensity of the patient as categorized as Low Activity Intensity during the diurnal portion of the day,
- *MAI_day:* fraction of the moments when the activity intensity of the patient is categorized as Moderate Activity Intensity during the diurnal portion of the day,
- *phasic_eda_night:* mean phasic EDA activity over the patient's sleeping period, the sleeping period being detected by the sleep/wake detector,
- *tonic_eda_night:* mean tonic EDA activity over the patient's sleeping period,
- *after_dinner_hr:* mean value of Heart Rate during a certain time period after the cessation of social activities, said time period being determined based on the patient's habit and/or clinical expertise, for instance between 7pm and 8pm,
- *after_dinner_LAI:* fraction of the moments when the patient's activity intensity is categorized as Low Activity Intensity during the above defined time period, such as between 7pm and 8pm,
- *after_dinner_MAI:* fraction of moments when

the patient's activity intensity is categorized as Moderate Activity Intensity during the same above defined time period,

◦ *after_dinner_VAI:* fraction of the moments when the patient's activity intensity is categorized as Vigourous Activity Intensity during the same above defined time period,

◦ *sedentary_after_dinner:* fraction of the moments when the patient's activity intensity is categorized as Sedentary during the same above defined time period,

◦ *sleep_regularity:* standard deviation of the daily sleep time over a period of several days or weeks, for instance 21 consecutive days,

◦ *sleep_time:* total duration between sleep onset and wake onset during the current night, as detected by a sleep detector such as the above-described sleep detection Hidden Markov Model,

◦ *waso_time:* total time spent awake between sleep onset and wake onset during the current night,

◦ *waso_regularity:* standard deviation of waso time, over multiple days, or weeks, for instance 21 consecutive days,

◦ *cardiac_amplitude:* difference between the mean heart rate value during a given time after sleep has begun, e.g., the first hour of sleep, and a given time before wake-up, e.g., the last hour of sleep,

◦ *respiratory_amplitude:* difference in the breathing rate value between a given time after sleep has begun, e.g., the first hour of sleep, and a given time before wake-up, e.g., the last hour of sleep,

◦ *LAI_night:* During the current night, that is, between sleep onset and wake onset, the fraction of time categorized as Low Activity intensity,

◦ *MAI_night:* During the current night, that is, between sleep onset and wake onset, the fraction of time categorized as Moderate Activity intensity,

◦ *VAI_night:* During the current night, that is, between sleep onset and wake onset, the fraction of time categorized as Vigourous Activity intensity,

◦ *REM_cycles:* number of REM phases during the current night,

◦ *REM_cycles_regularity:* standard variation of the number of REM phases during the night over a period of several days or weeks, for instance 21 consecutive days,

◦ *Sleep_efficacy:* total time spent sleeping divided by the duration of the patient's night duration, that is, (sleep_time - waso_time)/sleep_time.

*Sleep_efficacy* may also be defined as the total sleep time divided by the total time in bed if the latter can be obtained using a wearable device.

• *std_breathing_rate_night:* standard deviation of n_steps over multiple days or weeks, for instance 21 days,

• *diff_hr_before_after_sleep_onset:* the difference between the mean HR value during the hour before and the hour after sleep onset (in BPM),

• *REM_difference_night:* difference between the total time of REM phases during the first hour and last hour of sleep (in hours),

• *wake_onset:* time when the patient wakes up that day (in hours),

• *total_nap_time:* sum of all diurnal sleeping periods (in hours).

Grouping into feature sets

The signal processing module 2 may be configured to directly extract the pre-selected features $\{f_s\}$ from the signal S(t) and to group them into pre-defined feature sets.

The grouping strategy may be determined beforehand by an expert of the field for each type of mental disorder, for instance, for patient with MDD, by a clinician knowing the main symptoms of MDD.

In other embodiments, the grouping strategy may be determined by processing historical data collected on the patient using an optimization algorithm known in the art. For instance, the selection and grouping of features may be determined as to maximize the accuracy of a main task of interested, using any adapted optimization method such as a grid search.

As shown in figure 2, the 28 above-described features $\{f_s\}$ are distributed into 4 feature sets $\{X_1(t), X_2(t), X_3(t), X_4(t)\}$, each feature set corresponding to a main symptom of major depression disorder: sleep quality, diurnal activity, psychomotor retardation and anxiety.

In particular, in the example considered, the features are distributed as follows:

- the **sleep quality feature set $X_1(t)$** comprises features related to the activity and/or the sleep phases and/or the sleep and wake periods and/or the heart rate and/or breathing rate of the subject during the night, for example:
  *sleep_regularity, sleep_time, waso_regularity, waso_time, cardiac_amplitude, respiratory_amplitude, LAI_night, MAI_night, VAI_night, REM_cycles, REM_cycles_regularity, sleep efficacy, REM_difference_night, wake_onset, diff_hr_before_after_sleep_onset, std_breathing_rate_night* (16 features),
- the **diurnal activity feature set $X_2(t)$** comprises features related to the daily number of steps of the subject and/or the daily activity of the subject, such as:
  *n_steps, n_steps_regularity, sedentary_day, LAI_day, MAI_day, total_nap_time (6* features),
- the **psycho-motor retardation feature set $X_3(t)$** comprises features related to the activity and/or the heart rate of the subject during the first moments after wake-up, such as:
  *fhawo_LAI, fhawo_MAI, fhawo_VAI, fhawo_hr_high, fhawo_hr high_regularity* (5 features), and,

- the **anxiety feature set $X_4(t)$** comprises features related to the EDA activity over the subject's sleeping period, and/or activity and heart rate of the subject during a daily period after cessation of social activities, such as:

  *phasic_eda_night,          tonic_eda_night,*
  *after_dinner_hr,          after_dinner_LAI,*
  *after_dinner_MAI,          after_dinner          VAI,*
  *sedentary_after_dinner* (7features).

➢ Hierarchical scoring

The feature sets $\{X_1(t), X_2(t), X_3(t), X_4(t)\}$, once outputted by the signal processing module 2, are fed to the hierarchical scoring module for generating the predictions of both the sub-scores $\{y_1(t),y_2(t),y_3(t),y_4(t)\}$ and the global score *Y(t)*.

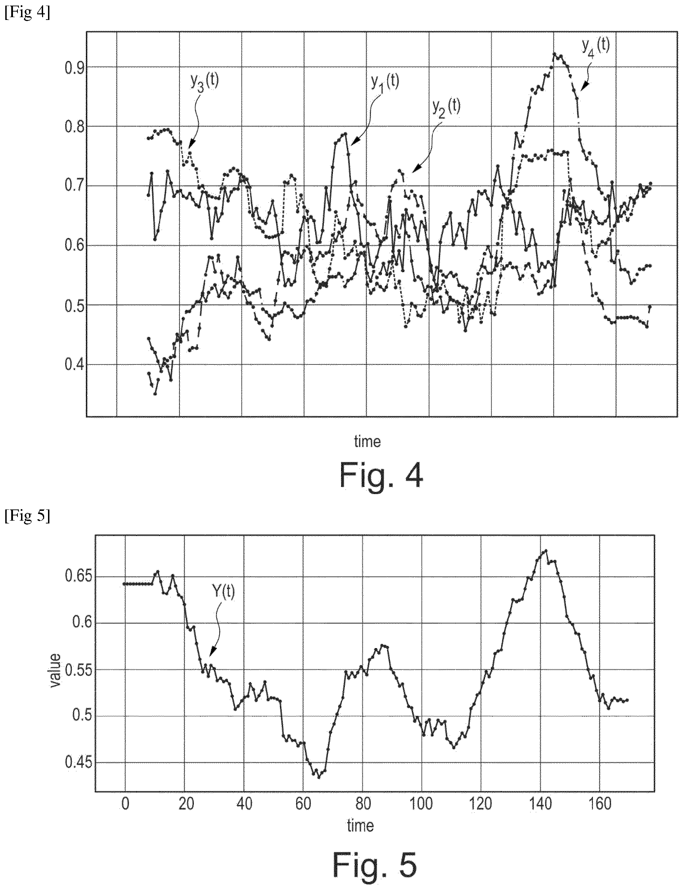

As illustrated in figure 4 for an exemplary patient, the evolution of sub-score $y_i(t)$ may be seen as the evolution of the depressive characteristic represented by the feature set $X_i(t)$ over the period of time over which data was collected in the first place.

Accordingly, over that monitored period of time:

- $y_1(t)$ represents the evolution of the sleep quality of the patient,
- $y_2(t)$ represents the evolution of the diurnal activity of the patient,
- $y_3(t)$ represents the evolution of the psychomotor retardation of the patient, and
- $y_4(t)$ represents the evolution of the anxiety of the patient.

The sub-scores $\{y_1(t),y_2(t),y_3(t),y_4(t)\}$ may then be aggregated together into a global score Y(t), as illustrated in figure 5. The global score *Y(t)* may be interpreted as an indicator of the daily evolution of the disease's severity in the patient.

In particular, the system according to the invention is able to evaluate over the period of time considered whether the patient is currently relapsing or recovering from the disease.

Moreover, since the global score *Y(t)* may be computed on a daily basis (or even more frequently), the evolution of the disease's severity may be predicted more precisely than the MADRS assessed by the clinician during visits, and the system according to the invention may help discover hidden events that may occur in-between the visits.

An exemplary training method for the neural network NN of the hierarchical module 4 is described below with reference to figures 6 and 7.

The neural network NN of the hierarchical module is trained to learn simultaneously to evaluate the temporal sub-scores over the monitoring period and the aggregation formula for computing a temporal global score from said temporal sub-scores.

As illustrated in figure 6, during the training phase, the neural network NN of the hierarchical module 4 uses empirical labels $\{E_j\}$ as additional input, along with the feature sets $\{X_1(t), X_2(t), X_3(t), X_4(t)\}$ comprising the continuous extracted features on the patient.

As described above, said empirical labels $\{E_j\}$ may comprise patient assessments using a clinical scale. In the example considered, the clinical labels are the above-mentioned MADRS scores $\{E_j\}$ assessed by a clinician during the patient's visits. Each MADRS score is determined to be representative of the actual mental state of the patient over a period of validity of about 5 days before and after the visit.

However, as described above, such empirical data is usually sparse and irregular, and may not cover continuously the period of time over which the prediction is to be generated.

In the example considered, there are seven clinical visits over a period of six months, with on average a visit every 30 days, which yields a set of seven MADRS scores $\{E_1, ..., E_7\}$.

As illustrated in figure 6 and in figure 7, pseudo-labels $E(t)$, referred to as "physiological MADRS" in the example considered, may be computed from the set of MADRS scores $\{E_1, ..., E_7\}$ to fill the "gaps" between two periods of validity.

In the example considered, the physiological MADRS $E(t)$ is generated using a self-supervised algorithm known in the art trained to propagates the clinical labels $\{E_1, ..., E_7\}$ while trying to preserve at best the relationship physiology / MADRS score.

The global score computed by the NN is representative of the mental state of the patient at time t. The training and validation thus use a performance criterion based on an estimation error between the estimated global score and the pseudo-evaluation labels.

In the example considered, the neural network NN of the hierarchical module is trained using a training scheme compatible with learning sub-scores while getting information about multiple patients at once.

The algorithm understands the relationship between physiology and clinical evaluation for each patient and optimizes a universal formula that encapsulates all patients' specificities. This allows to reduce the period of time of training dataset.

The validation may comprise a Leave One Patient Out (LOPO) validation scheme, so that the algorithm is robust to new unseen patients.

➢ Forecasting

The four predicted sub-scores $\{y_1(t),y_2(t),y_3(t),y_4(t)\}$ are processed through the forecasting module 6, which comprises a processing module 60 for generating a prediction of the evolution of each sub-score in the future.

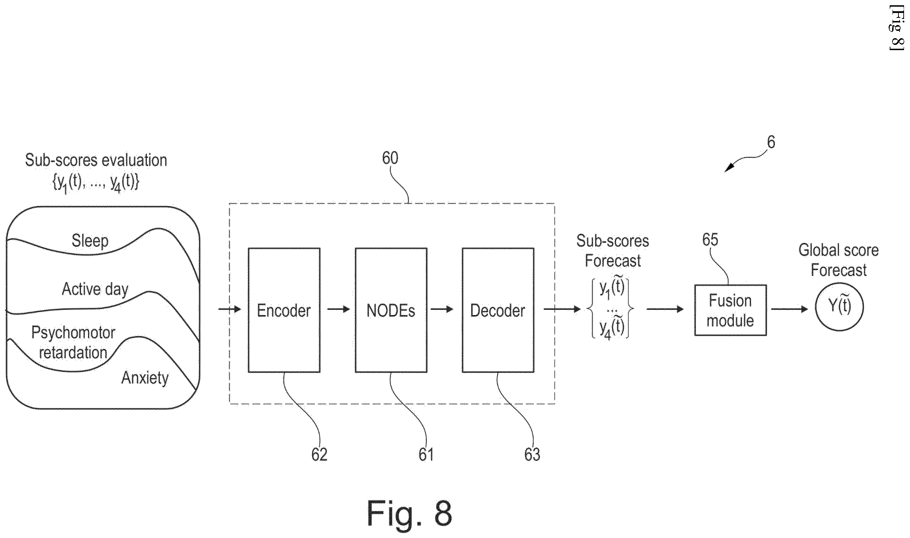

The processing module 60 uses a ML-based algorithm learning an ordinary differential equation (ODE) modeling the evolution of the feature sets over time. As illustrated in figure 8, the architecture of the algorithm may comprise an encoder 62 and NODEs 61.

In the example considered, the encoder 62 encodes the inputs, that is, the sub-scores $\{y_1, y_2, y_3, y_4\}$ into a plurality of latent variables $\{l_1, l_2, ..., l_n\}$ or a gaussian distribution of probability in the latent space, given by its mean and standard deviation. One can then sample a point from this gaussian distribution.

Each latent variable may capture hidden interactions between the sub-scores. The latent variables are fed to the NODEs 61 as a single input vector $V(t) = [l_1(t), l_2(t), ..., l_n(t)]$, the NODEs being trained beforehand for learning an ordinary differential equation modeling the variations $\dfrac{dV(t)}{dt}$ of the vector V with time.

In other embodiments, both the sub-scores and the latent variables may be fed to the NODEs.

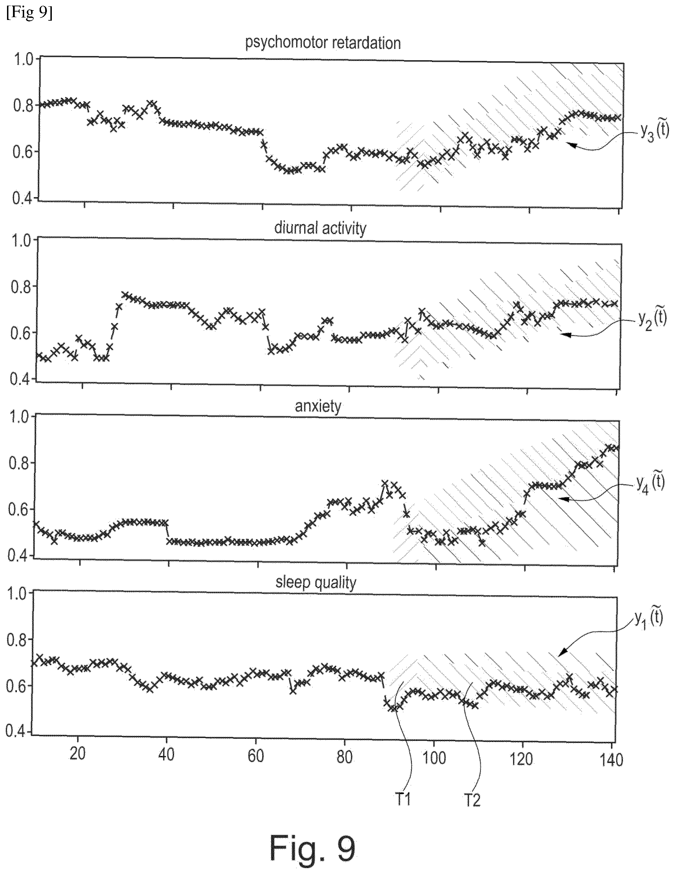

The output of the NODEs may then be transformed into a forecasted trajectory $\{y_1(\tilde{t}), ..., y_N(\tilde{t})\}$ for the sub-scores by a decoder 63, as illustrated on figure 9.

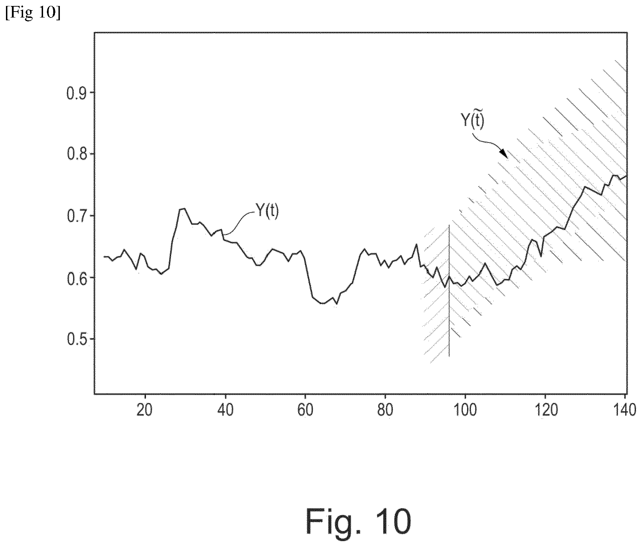

The forecasted sub-scores are then fused into a forecasted global score Y by the fusion module 65, as shown in Figure 10. The forecasted global score Y) may be seen as an indicator of the future evolution of the disease's severity in the patient.

Preferentially, one samples multiple points in the latent space from the encoded gaussian probability distribution to generate multiple likely evolution curves using the NODEs 61. In the example represented in figure 10, an input trajectory is encoded as a Gaussian probability in the latent space of the latent ODE model. Following up decoding, multiple samples 110 are drawn from the gaussian distribution. The samples T1 are used as initial conditions for the ODE which is solved by the NODEs. In that case, the outcome of the NODEs is not a single prediction (that is, a single plot per sub-score) but a distribution of predictions T2 in the feature space.

The perturbations of the latent variables may be useful to estimate the probability for critical events related to the disease to occur in the future; for instance, the probability for a patient with MDD of relapsing or being in remission within a particular period may be evaluated using this method and help the clinician in his decision-making process.

The perturbations may also be useful for estimating the disorder recurrence for a particular patient.

"Relapse" refers here to a depression relapse, where clinical signs of the disease appear again after a remission period, but before the patient is cured.

"Remission" may be partial or complete. A partial remission indicates that the patient does no longer show enough clinical signs to be diagnosed as suffering from MDD, but sparse symptoms may still be present. A complete remission refers to a patient no longer showing any sign of depression.

A patient may be considered cured after being in complete remission for a long enough time.

By "disorder recurrence", it is meant that a new major depressive disorder appears at least two months after the last episode.

The perturbations strategy is statistically relevant when the training of the NODEs is done using the ELBO loss, as for instance described in the above-mentioned publication from Rubanova, Yulia et al. "Latent odes for irregularly-sampled time series." (arXiv preprint arXiv:1907.03907 (2019)).

Although the presently disclosed subject matter and its advantages have been described in detail, it should be understood that various changes, substitutions, and alterations can be made herein without departing from the spirit and scope of the application as defined by the appended claims.

Moreover, the scope of the present application is not intended to be limited to the particular embodiments of the process, machine, manufacture, composition of matter, means, methods and steps described in the specification. As one of ordinary skill in the art will readily appreciate from the disclosure of the presently disclosed subject matter, processes, machines, manufacture, compositions of matter, means, methods, or steps, presently existing or later to be developed that perform substantially the same function or achieve substantially the same result as the corresponding embodiments described herein can be utilized according to the presently disclosed subject matter. Accordingly, the appended claims are intended to include within their scope such processes, machines, manufacture, compositions of matter, means, methods, or steps.

Hence, the invention is not limited to the examples that have just been described.

In particular, the ML algorithms of the different entities may comprise a different architecture, or may not be neural networks but other types of ML algorithms

The system and forecasting methods according to the invention is not limited to the mental state of a patient suffering from MDD.

In particular, as described above, it may be applied to patients suffering from post-traumatic stress disorder (PTSD). For this application, similar feature sets may be obtained from the sensing signals, since it is known that PTSD and MDD have an average of 60% overlap.

Other types of empirical labels may be used for training the at least one ML algorithm of the hierarchical scoring module. For instance, the empirical assessments for PTSD may be the classical CAPS-5 score, or any other label using a clinical scale suited for this mental disorder.

The system according to the invention may be applied, as described above, to any other type of dynamical systems that are empirically assessed by a human, using data that is possibly outside of the scope of the measurements, healthcare-related or not.

Other types of feature sets can be used and different combinations with the above-described feature sets may be tested.

**Claims**

1. A computer-based forecasting system for generating a prediction of the evolution of a mental state of a living subject likely to suffer from a mental disorder, the prediction being generated from time-dependent signals acquired beforehand on the subject, the system comprising:

   a. A signal processing module for generating, from the time-dependent signals, at least two feature sets comprising time-series of a plurality of extracted features, each feature set relating to a symptom of the mental disorder,
   b. A hierarchical scoring module comprising at least one predictor comprising at least one machine learning (ML) algorithm for processing the at least two feature sets and generating for each feature set, an evaluation of a respective temporal sub-score, and,
   c. A forecasting module comprising a processing module for processing the evaluated temporal sub-scores through at least one machine learning (ML) algorithm and generating a prediction of the evolution of at least one indicator related to the mental state of the subject and/or of the mental state of the subject.

2. The system of claim 1, wherein the at least one machine learning algorithm of the forecasting module comprises a neural network (NN), preferentially Neural ODEs (NODEs), a temporal one-dimensional convolutional neural network (1D CNN) or a recurrent neural network (RNN).

3. The system of claim 1, wherein the at least one ML algorithm of the forecasting module is configured to generate a forecast for each temporal sub-score.

4. The system of claim 3, wherein the forecasting module comprises a fusion module for aggregating together the forecasts of the sub-scores and computing the forecast of a global score representative of the mental state of the subject.

5. The system of claim 1, comprising a user-interface for informing a user of the at least one indicator related to the mental state of the subject and/or of the mental state of the subj ect.

6. The system of claim 1, wherein the mental disorder comprises a mood disorder and/or an anxiety disorder.

7. The system of claim 1, wherein the mental disorder comprises major depression disorder (MDD) or post-traumatic stress disorder (PTSD).

8. The system of claim 1, wherein the hierarchical module comprises a fusion module for aggregating together at least two evaluated temporal sub-scores and computing a global temporal score representative of the mental state of the subject.

9. The system of claim 1, wherein the time-dependent signals comprise time series of physiological measurements.

10. The system of claim 9, the physiological measurements comprising one or more among electro-cardiogram (ECG), body temperature, photoplethysmogram (PPG), activity records (ACC), electrodermal activity (EDA).

11. The system of claim 1, the plurality of extracted features comprising data related to actimetry, sleep and wake periods, sleep phases and/or step counting, cardio-respiratory autonomous nervous system and/or heart rate variability (HRV).

12. The system of claim 1, the feature sets comprising one or more sleep quality, anxiety, psychomotor retardation, and diurnal activity, preferably all of them.

13. The system of claim 1, the feature sets comprising:

   - an anxiety feature set comprising features relating to the EDA activity over the subject's sleeping period, and/or activity and heart rate of the subject during a daily period after cessation of social activities,
   - a psychomotor retardation feature set comprising features relating to the activity and/or the heart rate of the subject during the first moments after wake-up,
   - a diurnal activity feature set comprising features relating to the daily number of steps of the subject and/or the daily activity of the subject, and
   - a sleep quality feature set comprising features relating to the activity and/or the sleep phases and/or the sleep and wake periods and/or the heart rate and/or breathing rate of the subject during the night.

14. The system of claim 1, wherein the at least one ML algorithm of the predictor comprises an interpretable machine learning algorithm comprising one or more among logistic regression, shallow multi-layer perceptron or Neural 2-Choquet integrals.

15. The system of claim 1, wherein the ML algorithm of the hierarchical scoring module is a Neural 2-Choquet integrals network.

16. The system of claim 1, wherein the processing module comprises:

 - an encoder for building a point or a distribution of probability in a latent space using the temporal sub-scores, and
 - neural ODEs predicting the evolution of said point or said distribution of probability and/or the evolution of the temporal sub-scores using an ordinary differential equation.

17. The system of claim 1, wherein the at least one ML algorithm of the hierarchical scoring module is trained beforehand using a training dataset collected from multiple living subjects.

18. The system of claim 1, comprising an acquisition device sensing the subject for generating said time-dependent signals, the acquisition device comprising at least one of an accelerometer, an optical sensor, a heart rate detector and an electrodermal activity sensor.

19. A computer-implemented method for forecasting the evolution of a mental state of a living subject likely to suffer from a mental disorder, the method comprising:

 a) Acquiring during a monitoring period between a first and a second date time-dependent signals from at least one acquisition device sensing the subject,
 b) Generating at least two feature sets comprising time-series of a plurality of features extracted from these signals, each feature set relating to a symptom of the mental disorder,
 c) Using at least one ML algorithm for generating, for each feature set, an evaluation of a respective temporal sub-score between said first and second date,
 d) Using at least one ML algorithm to process the evaluated temporal sub-scores and generate a prediction of the evolution of a least one indicator related to the mental state of the subject and/or of the mental state of the subject.

20. The method of claim 19, comprising computing at step c), with the at least one ML algorithm, a global temporal score from the temporal sub-scores between said first and second date, the global score being representative of the mental state of the subject between said first and second date.

21. The method of claim 19, comprising generating with the at least one ML algorithm at step d) a forecast for each temporal sub-score.

22. The method of claim 21, comprising aggregating together the forecasts of the sub-scores and computing the forecast of a global score as an indicator of the mental state of the subject.

23. The method of claim 19, comprising at step d), encoding a point or a distribution of probability in a latent space representative of interactions between the symptoms from the sub-scores before processing them with the at least one machine learning algorithm.

24. The method of claim 19, comprising encoding a gaussian distribution probability in the latent space to draw multiple latent points from it, or encoding a latent point and perturbing it, to estimate the probability for an event related to the mental disorder to occur in a given time frame.

25. A method for training a ML algorithm to compute at least two temporal sub-scores for the evaluation of a mental state of a living subject likely to suffer from a mental disorder, each temporal sub-score being an indicator of the state of a symptom of the mental disorder, the method comprising:

 • Generating a learning dataset using data obtained from a plurality of living subjects through, for each subject:

  ◦ acquiring during a monitoring period, time-dependent signals from at least one acquisition device sensing the subject,
  ◦ generating at least two feature sets comprising time-series of a plurality of features extracted from these signals, each feature set being associated to a respective temporal sub-score to be evaluated,
  ◦ prompting a user to input clinical evaluation labels relating to the clinical evaluation of the mental state of said subject at respective visit dates during the monitoring period, and
  ◦ using a ML algorithm to generate pseudo-evaluation labels at additional dates between the visit dates, each representative of an assessed mental state of the patient at a corresponding additional date,

 • Using said learning dataset for training the ML algorithm to estimate, for each subject, the temporal sub-scores over the monitoring period,

  the ML algorithm being trained simultaneously to aggregate the temporal sub-scores into a temporal global score over the monitoring period,

said joint training using a performance criterion based on a comparison between said temporal global score with the pseudo-evaluation labels.

26. The method of claim 25, the neural network comprising a hierarchical 2-Choquet integral network.

[Fig 1]

Patient P

$S(t)$

4

6

1

Feature sets

sub-scores prediction

Processing module

sub-scores forecast

Signal processing module

$\{X_1, ..., X_N\}$

Predictor

NN

$\{y_1(t), ..., y_N(t)\}$

NODEs

$\{y_1(\tilde{t}), ..., y_N(\tilde{t})\}$

2

41    40

Fusion module

60

Fusion module

45

Global score prediction

65

**Global score forecast**

$Y(t)$

$Y(\tilde{t})$

# Fig. 1

[Fig 2]

Patient P

D

$S(t)$

Clinical expertise

2

20

**Grouping into feature set**

Sleep quality

Diurnal activity

Psychomotor Retardation

Anxiety

**Raw data collection**
Physiological signals $S(t)$

**Feature extraction**
N1 features extracted
$\{f_e\}$

**Feature selection**
N2 features selected
$\{f_s\}$

21

22

$\{X_1, X_2, X_3, X_4\}$

# Fig. 2

[Fig 3]

Fig. 3

[Fig 4]

time

Fig. 4

[Fig 5]

Fig. 5

[Fig 6]

Fig. 6

[Fig 7]

Fig. 7

[Fig 8]

Fig. 8

[Fig 9]

Fig. 9

[Fig 10]

Fig. 10

Europäisches
Patentamt

European
Patent Office

Office européen
des brevets

**EUROPEAN SEARCH REPORT**

Application Number

EP 22 30 5114

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2021 0068697 A (SHIN MIN KYUE [KR]; PARK KANG HYUN [KR] ET AL.) 10 June 2021 (2021-06-10) * paragraph [0006] * * paragraph [0022] – paragraph [0023] * * paragraph [0028] – paragraph [0032] * | 1-15 | INV. G06N3/04 G16H50/30 G16H50/50 |
| X | US 2018/342326 A1 (MOTURU SAI [US] ET AL) 29 November 2018 (2018-11-29) * paragraph [0015] * * paragraph [0025] * * paragraph [0037] – paragraph [0045] * * paragraph [0068] – paragraph [0069] * * figures 4, 6 * | 1-15 | |
| A | WO 2022/006183 A1 (RESMED PTY LTD [AU]; RESMED INC [US] ET AL.) 6 January 2022 (2022-01-06) * paragraph [0204] – paragraph [0224] * * figure 13 * | 1-15 | |

TECHNICAL FIELDS
SEARCHED        (IPC)

G06N
G16H

The present search report has been drawn up for all claims

| Place of search | Date of completion of the search | Examiner |
|---|---|---|
| Munich | 19 October 2022 | Hauber, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 30 5114

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2022

| Patent document cited in search report | | Publication date | Patent family member(s) | | Publication date |
|---|---|---|---|---|---|
| KR 20210068697 | A | 10-06-2021 | NONE | | |
| US 2018342326 | A1 | 29-11-2018 | US | 2015370993 A1 | 24-12-2015 |
| | | | US | 2018342326 A1 | 29-11-2018 |
| | | | US | 2022059235 A1 | 24-02-2022 |
| WO 2022006183 | A1 | 06-01-2022 | WO | 2022006181 A1 | 06-01-2022 |
| | | | WO | 2022006182 A1 | 06-01-2022 |
| | | | WO | 2022006183 A1 | 06-01-2022 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FOMPEYRINE et al.** Enhancing human-machine teaming for medical prognosis through neural ordinary differential equations (NODEs). *Human-Intelligent Systems Integration,* 2021
- **BRESSON, ROMAN et al.** Neural representation and learning of hierarchical 2-additive Choquet integrals. *Proceedings of the Twenty-Ninth International Conference on International Joint Conferences on Artificial Intelligence.,* 2021
- **RUBANOVA, YULIA et al.** Latent odes for irregularly-sampled time series. *arXiv:1907.03907,* 2019
- **BRESSON, ROMAN et al.** Neural representation and learning of hierarchical 2-additive Choquet integrals. *Proceedings of the Twenty-Ninth International Conference on International Joint Conferences on Artificial Intelligence,* 2021
- **MENAI, MEHDI et al.** Accelerometer assessed moderate-to-vigorous physical activity and successful ageing: results from the Whitehall II study. *Scientific reports,* 2017, vol. 7 (1), 1-9
- **OTZENBERGER, HELENE et al.** Dynamic heart rate variability: a tool for exploring sympathovagal balance continuously during sleep in men. *American Journal of Physiology-Heart and Circulatory Physiology,* 1998, vol. 275 (3), H946-H950
- **THOMAS, ROBERT JOSEPH et al.** An electrocardiogram-based technique to assess cardiopulmonary coupling during sleep. *Sleep,* 2005, vol. 28 (9), 1151-1161
- **ROEBUCK, A. et al.** A review of signals used in sleep analysis. *Physiological measurement,* 2013, vol. 35 (1), R1
- **RUBANOVA, YULIA et al.** Latent odes for irregularly-sampled time series. *arXiv:1907.03907,* 2019